(19) Europäisches Patentamt
European Patent Office
Office européen des brevets

(11) Veröffentlichungsnummer: **0 414 175 A2**

# (12) EUROPÄISCHE PATENTANMELDUNG

(21) Anmeldenummer: **90115869.1**

(22) Anmeldetag: **18.08.90**

(51) Int. Cl.[5]: **A61K 31/52**

Die Bezeichnung der Erfindung wurde geändert (Richtlinien für die Prüfung im EPA, A-III, 7.3).

(30) Priorität: **22.08.89 DE 3927596**
**12.09.89 DE 3930392**

(43) Veröffentlichungstag der Anmeldung:
**27.02.91 Patentblatt 91/09**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Hänel, Heinz, Dr.**
**Taunusstrasse 148**
**D-6370 Oberursel(DE)**
Erfinder: **Schrinner, Elmar, Dr.**
**Hedwigstrasse 2**
**D-6200 Wiesbaden(DE)**
Erfinder: **Schmidts, Hans-Ludwig, Dr.**
**Adlerflychtstrasse 4**
**D-6000 Frankfurt am Main(DE)**

## (54) Verwendung von Xanthinderivaten zur Nephrotoxizitätsverminderung.

(57) Xanthinderivate verringern die Nephrotoxizität von Stoffwechselprodukten und nephrotoxischen Arzneimittelbestandteilen.

EP 0 414 175 A2

## VERWENDUNG VON XANTHINDERIVATEN SOWIE ARZNEIMITTEL ENTHALTEND EIN XANTHINDERIVAT ZUR NEPHROTOXIZITÄTSVERMINDERUNG

Die vorliegende Erfindung betrifft die Verwendung von Xanthinderivaten sowie Arzneimittel enthaltend ein Xanthinderivat zur Nephrotoxizitätsverminderung.

Es ist bereits bekannt, daß die Nephrotoxizität von Arzneimittelwirkstoffen durch die gleichzeitige Verabreichung von NaCl gesenkt werden kann (vgl. J. Cancer Res. Clin. Oncol. 114, Suppl., S. 158, 1988). Weiterhin wurde bereits vorgeschlagen, Arzneimittel, die eine erhöhte Freisetzung der Tumor Nekrosis Faktor (TNF) bewirken, mit einem TNF-Inhibitor, z.B. Xanthinderivaten, zu kombinieren (vgl. EP O 344 586).

Überraschenderweise wurde nun gefunden, daß bestimmte Xanthinderivate die Nephrotoxizität von Arzneimittelwirkstoffen und Stoffwechselprodukten senken können. Erfindungsgegenstand ist demzufolge die Verwendung eines Xanthinderivats zur Senkung der Nephrotoxizität von Arzneimittelwirkstoffen und Stoffwechselprodukten.

Bevorzugte Xanthinderivate stammen aus der Gruppe der folgenden Verbindungen

1) Verbindungen der Formel I

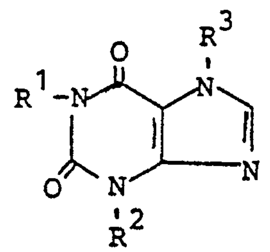

(I)

in der einer der Reste $R^1$ und $R^3$ eine geradkettige Alkyl-, ($\omega$-1)-Oxoalkyl- oder ($\omega$-1)-Hydroxyalkylgruppe mit 3 bis 8 C-Atomen und die beiden anderen Reste $R^2$ und $R^3$ oder $R^1$ und $R^2$ geradkettige oder verzweigte Alkylgruppen mit 1 bis 8 C-Atomen in der Position von $R^1$ und $R^3$ und 1 bis 4 C-Atomen in der Position von $R^2$ darstellen, wobei die Summe der C-Atome dieser beiden Alkylsubstituenten höchsten 10 beträgt,

2) Verbindungen der Formel II

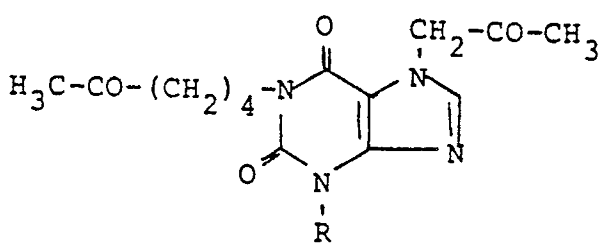

(II)

in der R für einen Alkylrest mit 1 bis 4 C-Atomen steht,

3) Verbindungen der Formel III

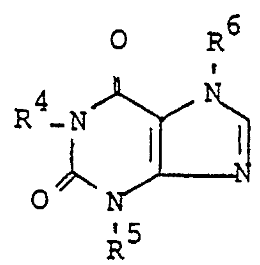

(III)

in der mindestens einer der Reste $R^4$ und $R^6$ eine tertiäre Hydroxyalkylgruppe der Formel

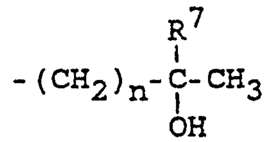

(IIIa)

darstellt, wobei $R^7$ eine Alkylgruppe mit bis zu 3 C-Atomen und n eine ganze Zahl von 2 bis 5 bedeuten, und - falls nur einer der Reste $R^4$ oder $R^6$ eine solche tertiäre Hydroxyalkylgruppe der Formel IIIa bedeutet - der andere Rest für ein Wasserstoffatom oder einen aliphatischen Kohlenwassertoff-Rest $R^8$ mit bis zu 6 C-Atomen steht, dessen Kohlenstoffkette von bis zu 2 Sauerstoffatomen unterbrochen oder mit einer Oxogruppe oder bis zu zwei Hydroxygruppen substituiert sein kann (wobei eine im Rest $R^8$ befindliche Oxo- oder Hydroxygruppe vorzugsweise durch mindestens 2 C-Atome vom Stickstoff getrennt ist), und $R^5$ eine Alkylgruppe mit 1 bis 4 C-Atomen darstellt,

4) Prodrug-Formen der Verbindungen der Formeln I bis III und/oder

5) Metabolite der Verbindungen der Formeln I bis III.

Unter diesen Verbindungen sind wiederum jene der Formel I besonders bevorzugt, die in der Position von $R^1$ oder $R^3$ eine Hexyl-, 5-Oxohexyl- oder 5-Hydroxyhexylgruppe tragen. Zu ihnen gehören insbesondere

1-Hexyl-3,7-dimethylxanthin,
1-(5-Hydroxyhexyl)-3,7-dimethylxanthin,
3,7-Dimethyl-1-(5-oxohexyl)-xanthin,
7-(5-Hydroxyhexyl)-1,3-dimethylxanthin,
1,3-Dimethyl-7-(5-oxohexyl)-xanthin,
1-(5-Hydroxyhexyl)-3-methyl-7-propylxanthin und
3-Methyl-1-(5-oxohexyl)-7-propylxanthin (= Propentofyllin),
vor allem 3,7-Dimethyl-1-(5-oxohexyl)xanthin (= Pentoxifyllin).

Besonders bevorzugte Verbindungen der Formel III sind solche Verbindungen, bei denen $R^5$ für eine Methyl- oder Ethylgruppe steht. Gleichermaßen bevorzugt sind jene Verbindungen der Formel III, in denen nur einer der beiden Reste $R^4$ oder $R^6$ die vorstehend definierte tertiäre Hydroxyalkylgruppe darstellt. Weiterhin bevorzugt sind solche Verbindungen, bei denen $R^7$ für eine Methylgruppe steht und n eine ganze Zahl von 3 bis 5 bedeutet, so daß der tertiäre Hydroxyalkylrest IIIa entweder [(ω-1)-Hydroxy-(ω-1)-methyl]-pentyl, -hexyl oder -heptyl darstellt, insbesondere solche, bei denen $R^5$ Methyl oder Ethyl bedeutet.

Weiterhin sind diejenigen Verbindungen der Formel III besonders hervorzuheben, in denen $R^4$ die tertiäre Hydroxyalkylgruppe darstellt und $R^6$ für Wasserstoff, Alkyl, Hydroxyalkyl oder Alkoxyalkyl mit jeweils 1 bis 4 C-Atomen steht, wie etwa das 7-Ethoxymethyl-1-(5-hydroxy-5-methylhexyl)-3-methylxanthin und das 1-(5-Hydroxy-5-methylhexyl)-3-methylxanthin.

Eine weitere Ausführungsform der Erfindung besteht darin, daß die Oxoalkylxanthine der Formel I und II bzw. die Hydroxyalkylxanthine der Formel I und III nicht per se, sondern in Prodrug-Form eingesetzt werden, aus der erst im Organismus die therapeutisch wirksamen Xanthinverbindungen mit den in Formeln I, II und III definierten Substituenten durch Biotransformation freigesetzt werden können. Hierfür kommen beispielsweise die acetalisierten Oxoalkylxanthine, in denen die Carbonylgruppen durch das Strukturelement der Formel IV

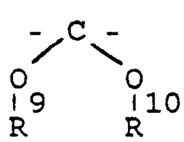

(IV)

ersetzt sind, und die O-acylierten Hydroxyalkylxanthine mit dem Strukturelement der Formel (V)

$R^{11}$-CO-O- (V)

anstelle der Hydroxyfunktion in Frage, wobei $R^9$ und $R^{10}$ eweils eine Alkylgruppe mit bis zu 4 C-Atomen oder zusammen eine Ethylen-, Trimethylen- oder Tetramethylengruppe darstellen und $R^{11}$ einen Alkylrest mit bis zu 4 C-Atomen oder gegebenenfalls substituiertes Phenyl oder Pyridyl bedeutet.

Die Herstellung von Xanthinderivaten ist z.B. in der DE-B-1 233 405, der DE-B-1 235 320 oder der DE 35 25 801 A1 beschrieben. Die Xanthinderivate sind geeignet bei unterschiedlichen Wirkstoffen und auch körpereigenen Stoffen wie z.B. Stoffwechselprodukten die Nephrotoxizität zu vermindern. Besonders ausgeprägt tritt die Senkung der Nephrotoxizität bei der Verabreichung des Amphotericin B in Erscheinung.

Die Xanthinderivate können sowohl allein, in Kombination mit dem nephrotoxischen Arzneimittelbestandteil in getrennten Dosierungseinheiten (gleichzeitigt oder in zeitlicher Abfolge) als auch mit dem nephrotoxischen Arzneimittelbestandteil vermischt verabreicht werden.

Die vorliegende Erfindung betrifft auch Arzneimittel, die dadurch gekennzeichnet sind, daß sie mindestens einen nephrotoxischen Bestandteil und mindestens ein Xanthinderivat zur Senkung der Nephrotoxizität enthalten.

Die genannten Verbindungen und Arzneimittel können auf unterschiedliche Weise appliziert werden. Z.B. können sie intravenös, intramuskulär, intraperitoneal, subkutan oder oral verabreicht werden.

Bei den erfindungsgemäßen Arzneimitteln kann des Verhältnis von nephrotoxischer Verbindung zu Xanthinderivat einen weiten Bereich überstreichen. Zu bevorzugen ist ein Gewichtsverhältnis von ca. 1:100 bis ca. 100:1, besonders bevorzugt von ca. 1:10 bis ca. 10:1.

Die Herstellung erfindungsgemäßer Arzneimittel, die ebenfalls zum Gegenstand der vorliegenden Erfindung gehört, erfolgt, indem die nephrotoxische Verbindung und mindestens ein Xanthinderivat gegebenenfalls mit weiteren Zusatz-und/oder Hilfsstoffen in eine geeignete Darreichungsform gebracht werden. Die Zusatz- oder Hilfsstoffe stammen aus der Gruppe der Trägermittel, Konservierungsstoffe und anderer üblicher Hilfsstoffe. Z.B. können für orale Darreichungsformen Hilfsstoffe wie Stärke, z.B. Kartoffel-, Mais- oder Weizenstärke, Cellulose bzw. deren Derivate, insbesondere mikrokristalline Cellulose, Siliziumdioxid, verschiedene Zucker wie Milchzucker, Magnesiumcarbonat und/oder Calciumphosphate verwendet werden. Weiterhin ist es vorteilhaft, den oralen Darreichungsformen Hilfsstoffe zuzusetzen, die die Verträglichkeit der Medikamente verbessern, wie z.B. Schleimbildner und Harze. Zwecks besserer Verträglichkeit können die Medikamente auch in Form von magensaftunlöslichen Kapseln verabreicht werden. Darüber hinaus kann es vorteilhaft sein, der Darreichungsform, bzw. einer Komponente des Kombinationspräparats, ein Retardierungsmittel zuzusetzen, gegebenenfalls in Form von permeablen Membranen, wie z.B. solche auf Cellulose- oder Polystyrolharzbasis, oder Ionenaustauscher.

Die jeweils tägliche Dosis des zu verabreichenden Xanthinderivats kann einen weiten Bereich überstreichen und hängt ab von dem Xanthinderivat und dem nephrotoxischen Mittel. Zu bevorzugen ist eine tägliche Xanthinderivatdosis von 1-20 mg/kg Körpergewicht, besonders bevorzugt von 1-10 mg/kg, insbesondere von 1-5 mg/kg.

Die nephrotoxizitätssenkende Wirkung von Xanthinderivaten konnte in folgender Weise experimentell nachgewiesen werden

Es wurden Mäuse im Verlauf von 11 Tagen 9 mal intravenös mit Pentoxifyllin (20 mg/kg), Amphotericin B (4 mg/kg) und Amphotericin B (4 mg/kg) in Kombination mit Pentoxifyllin (20 mg/kg) sowie mit einer Lösungsmittelkontrolle behandelt. Zur Beurteilung der Nephrotoxizität der jeweils verabreichten Substanzen wurden bei den Versuchstieren folgende Stoffe bestimmt:

Creatinin: Eine gestörte Creatininclearance durch eine geschädigte Niere führt zu einem erhöhten Gehalt von Creatinin im Serum (Hypercreatininaemie)

Harnstoff : Erhöhte Konzentrationen im Serum werden regelmäßig bei einer schweren Nierenfunktionsstörung beobachtet. Eine verminderte Harnstoffclearance führt zu einem erhöhten Gehalt im Serum

Kalium: Da Kaliumionen bis zu 95 % durch die Nieren ausgeschieden werden, ist eine Hyperkaliaemie ein deutliches Zeichen für eine schwere Nierenfunktionsstörung.

Tabelle 1

| Pathophysiologische Befunde im Serum von Mäusen. | | | | |
|---|---|---|---|---|
| 9 mal intravenös behandelt (11 Tage)<br>Meßwerte aus gepoolten Seren von je 3 Mäusen | | | | |
| Parameter | Lösungsmittelkontrolle | Pentoxifyllinkontrolle | Amphotericin B | Amphotericin B + Pentoxifyllin |
| Creatinin (µmol/l) | 26 | 35 | 55 | 30 |
| Harnstoff (mmol/l) | 6,1 | 7,5 | 10,5 | 8,2 |
| Kalium (mmol/l) | 8,2 | 8,5 | 10,1 | 8,5 |

Tabelle 2 zeigt die Mortalitäten bei unterschiedlichen Gruppen von Mäusen nach einer Beobachtungszeit von 42 Tagen.

Tabelle 2

| Mäuse, je 30 tägliche intravenöse Injektionen an je 5 aufeinanderfolgenden Tagen in 4 Blöcken mit je 2 behandlungsfreien Zwischentagen<br>Mortalitäten nach einer Beobachtungszeit von 42 Tagen<br>Amphotericin B 4 mg/kg<br>Xanthinderivate je 20 mg/kg<br>gelöst in 5 %iger wäßriger Glucoselösung | |
|---|---|
| Amphotericin B | 73 von 92 Mäusen gestorben |
| 3,7-Dimethyl-1-(5-oxohexyl)xanthin | 0 von 25 Mäusen gestorben |
| " + Amphotericin B | 7 von 25 Mäusen gestorben |
| 7-Ethoxymethyl-1-(5-hydroxy-5-methylhexyl)-3-methyl-xanthin | 0 von 25 Mäusen gestorben |
| " + Amphotericin B | 4 von 25 Mäusen gestorben |
| 1-(5-Hydroxy-5-methyl-hexyl)-3-methylxanthin | 0 von 25 Mäusen gestorben |
| " + Amphotericin B | 3 von 25 Mäusen gestorben |

Die Ergebnisse des beschriebenen Versuchs belegen die nephrotoxizitätssenkende Wirkung der Xanthinderivate.

Weiterhin läßt sich die nephrotoxizitätssenkende Wirkung der Xanthinderivate durch histologische Untersuchung von Versuchstieren feststellen.

Dazu wurden 56 Mäuse mit Glukose, Pentoxifyllin (20 mg/kg), Amphotericin B (4 mg/kg) oder der Kombination von Pentoxifyllin/Amphotericin B (20 mg/kg bzw. 4 mg/kg) über einen Zeitraum von 11, 25, 39 oder 53 Tagen intravenös behandelt.

Nach den angegebenen Versuchszeiten wurden je 4 Tiere getötet und Serum für serologische Untersuchungen entnommen. Die Nieren wurden in Bouin'schem Gemisch fixiert, nach üblicher histologischer Technik aufgearbeitet und PAS-gefärbt.

Die histopathologischen Untersuchungen zeigen, daß in der Pentoxifyllin-Gruppe nur ganz marginale Veränderungen zu finden sind, die sich von denen der Lösungsmittelkontrolle (Glukose) nur unwesentlich unterscheiden.

Im Gegensatz dazu weisen die Nieren der ausschließlich mit Amphotericin B behandelten Mäuse, die überlebt hatten, erhebliche Schäden des tubulären Apparats auf: So finden sich in frühen Phasen Nekrosen (möglicherweise überwiegend) distaler Tubuli mit Zeichen der Regeneration und in späteren Stadien Atrophien bevorzugt in Mark-nahen Rindenabschnitten, während sich vor allem proximale Nephronabschnitte infolge einer Abflußbehinderung weiten, hier z.T. Eiweißzylinder enthalten, druckatrophisch werden und - wie die distalen - mit einer Verbreiterung der Basalmembran reagieren. Im Bereich betroffener Nephrone treten makrophagozytäre Reaktionen und ausgeprägte, narbenartige interstitielle Bindegewebsvermehrungen auf. Für eine zusätzliche glomeruläre Beteiligung findet sich kein sicheres morphologisches Korrelat.

Im Gegensatz zu den mit Amphotericin-behandelten Tieren weist die Gruppe der kombinationsbehandelten insgesamt erheblich reduzierte Nierenschäden auf; dieses Phänomen läßt sich sowohl anhand der Intensität als auch des Spektrums der pathologischen Befunde demonstrieren, die beide gleichermaßen eingeschränkt sind.

Aus den o.g. Beobachtungen läßt sich schließen, daß im vorliegenden Modell die nephrotoxischen Eigenschaften von Amphotericin B durch die zusätzliche Gabe von Pentoxifyllin erheblich reduziert werden.

## Ansprüche

1. Verwendung eines Xanthinderivats zur Senkung der Nephrotoxizität von Arzneimittelwirkstoffen und Stoffwechselprodukten.
2. Arzneimittel, dadurch gekennzeichnet, daß es mindestens einen nephrotoxischen Bestandteil und mindestens ein Xanthinderivat zur Senkung der Nephrotoxizität enthält.
3. Arzneimittel, dadurch gekennzeichnet, daß es Amphotericin B und mindestens ein Xanthinderivat zur Senkung der Nephrotoxizität enthält.
4. Arzneimittel gemäß den Ansprüchen 2 oder 3, dadurch gekennzeichnet, daß das Xanthinderivat Pentoxifyllin und/oder Propentofyllin und/oder 1-(5-Hydroxy-5methylhexyl)-3-methylxanthin und/oder 7-Ethoxymethyl-1-(5-hydroxy-5-methylhexyl)-3-methylxanthin ist.

5. Verwendung eines Xanthinderivats zur Herstellung von Arzneimitteln zur Senkung der Nephrotoxizität eines Arzneimittelbestandteils.

6. Verwendung eines Xanthinderivats gemäß Anspruch 1 oder 5, dadurch gekennzeichnet, daß das Xanthinderivat Pentoxifyllin und/oder Propentofyllin und/oder 1-(5-Hydroxy-5-methylhexyl)-3-methylxanthin und/oder 7-Ethoxymethyl-1-(5-hydroxy-5-methylhexyl)-3-methylxanthin ist.

7. Verfahren zur Herstellung von Arzneimitteln gemäß Anspruch 2 oder 3, dadurch gekennzeichnet, daß mindestens ein nephrotoxischer Arzneimittelbestandteil und mindestens ein Xanthinderivat gegebenenfalls mit Zusatz- und/oder Hilfsstoffen in eine geeignete Darreichungsform gebracht wird.